Europäisches Patentamt

European Patent Office

Office européen des brevets

(19)

(11) **EP 1 195 177 A1**

## (12) EUROPEAN PATENT APPLICATION
published in accordance with Art. 158(3) EPC

(43) Date of publication:
**10.04.2002 Bulletin 2002/15**

(51) Int Cl.7: **A61N 5/10**, A61B 6/00,
G01N 23/04

(21) Application number: **00964808.0**

(22) Date of filing: **05.07.2000**

(86) International application number:
**PCT/RU00/00273**

(87) International publication number:
**WO 02/02188 (10.01.2002 Gazette 2002/02)**

(84) Designated Contracting States:
**AT BE CH CY DE DK ES FI FR GB GR IE IT LI LU MC NL PT SE**
Designated Extension States:
**AL LT LV MK RO SI**

(71) Applicant: **Kumakhov, Muradin Abubekirovich
Moscow, 123298 (RU)**

(72) Inventor: **Kumakhov, Muradin Abubekirovich
Moscow, 123298 (RU)**

(74) Representative: **Zellentin, Rüdiger
Patentanwälte
Zellentin & Partner
Zweibrückenstrasse 15
80331 München (DE)**

(54) **ROENTGEN DEVICE FOR LOCALIZATION AND RADIATION THERAPY OF CANCELLATION CANCERS**

(57)    In the first stage of a method of radiotherapy a location of a malignant neoplasm, emerged from the preceding diagnostics, is determined, for which purpose the part (7) of the patient's body (5), including the said neoplasm, is scanned. Scanning is carried out by moving a zone (4) of a radiation concentrating, formed by crossing of several X-ray beams. The information about a density of tissues is obtained by means of the detectors (6), a secondary radiation emerging in the concentrating zone (4) is transported to. In the second stage scanning is carried out by means of the same means as in the first stage, moving the concentrating zone in limits of the malignant neoplasm, determined in the first stage. Thus the X-ray sources (1) by means of a means of controlling (9) are reset to the regime of increased intensity, being sufficient for ray injury of the tissues of the malignant neoplasm. To deliver a radiation of the sources to the concentration zone and a secondary radiation to the detectors various combinations of the collimators, X-ray lenses (2, 3) and half lenses, forming together with the sources and detectors an X-ray optical system (8), are used. When the concentration zone is being scanned the X-ray optical system (8) as a whole and the patient's body (5) moves respectively (10a, 10b). The coordinate sensors (11), fixing the said moving, and the detectors (6) are connected with their outputs up to the means (12) of data processing and imaging. The usage of the said principle of obtaining the information about the density of biological tissues and common means for X-ray beams forming in both stages improves an accuracy of measuring and ray affect and decreases an irradiation doze of healthy tissues.

Fig.1

## Description

## Field of the invention

The inventions relate to the means of determining a malignant neoplasm in a patient's body and its treatment by means of an X-rays.

## Background art

The known methods involve carrying out a topometric preparation after a diagnosis is determined and a decision is made to use a radiation therapy of a malignant neoplasm with the use of an X-rays. During the said topometric preparation linear sizes, an area, a volume of pathologic forms, organs and anatomic structures are determined and their relative placement of a particular patient are described in quantitative terms (see, for example: Radiation therapy of a malignant neoplasm. Physicians guide. Ed. by prof. E.S. Kiseleva. Moscow, "Meditsina", 1996 [1], pp. 46-47). The main task of a topometric preparation is to combine various data obtaining from a diagnostics of a disease, and to give a radiologist all anatomical data about the area to be irradiated at 1:1 scale in the form, which makes possible to develop an irradiation program. It is necessary to know a form and sizes of a site-target, its location in the patient's body, as well as a syntopy of the surrounding tissues, a distance between the target and the most important anatomical structures and critical organs from the point of view of the radiation load propagation in order to choose variants and parameters of the irradiation program. The characteristic points and areas on the surface of the patient's body, with respect to which the X-rays are oriented subsequently when the irradiation takes place, are chosen, in particular, as a result of topometric preparation and the irradiation program developing.

The main disadvantage of the described combination of a patient preparing for an irradiation and an irradiation itself is that these stages are separated both in time and space, in particular because they are carried out by means of different means. An irradiation (ray action on the cells of a malignant neoplasm in order to hit them) is realized by means of the directional sources of rather powerful X-rays. Concerning X-ray researches preceding the irradiation, they are carried out at significantly lower intensity of a radiation and, besides, they can represent only one of the methods, applied in combination: angiography, excretion urography, gastrointestinal tract, bones of a skeleton and skull, and thorax researches; radionuclidal researches of bones and liver; ultrasonic methods (echoscopy, echotomography) for image formation of the organs of an abdominal cavity, pelvis, and soft tissues; computed tomography, which provides to form a high effective X-ray image; magnetic resonance tomography, etc. Therefore it is very difficult to obtain a high accuracy of the radiation action, and, as a result, either some parts of a malignant site are not irradiated, or an intensive X-rays concentrates in the area, exceeding the sizes of the said malignant site. If the latter is the case, the surrounding healthy tissues are irradiated significantly more, than the healthy tissues, which are on the radiation way to the malignant site.

When this method is realized, not only the errors of the reference points choosing and X-ray beams "directing" on the said points at the radiation action, but an inconstancy of the internal organs position, an inaccuracy of a patient placing at a radiation action at different sessions, as well. A radiation fractionation as itself, caused by the guest to avoid an overradiation of the healthy tissues, makes a vicious circle, as it is known, that a doze, delivered once to a malignant site and being sufficient for its irreversible injury, is in several times lower than a cumulative doze, being sufficient at the fractionation [1, pp. 84, 91].

To overcome this disadvantage special steps are taken in some known technical solutions, aimed at an accuracy increasing and a patient stable positioning (see, for example, USA patent No. 5,983,424, published 16.11.1999 [2]).

The usage of so called simulator, an X-ray diagnostic device being is quiet similar to the device for a distant irradiation by geometric and kinematic possibilities, is the other way to overcome the mentioned disadvantages [1, p. 55]. It is possible "to ray" a patient in different directions without changing his placement by means of the said simulator. At the topometric preparation a patient is placed on the table of the simulator in a position, which he will have at the irradiation session, and a roentgenoscopy is made. A center and borders of an irradiation volume are chosen, a plane, where a central axis of a radiation beam at a radiation action will be, is defined by means of a light cross and movable X-ray contrast fibers.

However none of such measures allow to avoid the errors of "directing" the beams, irradiating a malignant neoplasm, because these errors result from a tumor increasing. This factor becomes significantly efficient at a prolonged treatment, when the irradiation sessions are distanced in time from the moment of finalizing the diagnostic study of a patient.

Technical solutions, closest to the suggested inventions, are described in USA patent No. 5,207,223 (published 04.05.1993 [3]). According to this patent the images of the tissues structure of a patient are formed by means of the directed X-ray beams just before a radiation action and used for the correction of the irradiation program by comparing with the results of the preceding diagnostic researches. Thus, however, different beams are used to form the said images and the radiation action on the tissues of the malignant site, what makes impossible to escape errors in orientation of the irradiating beams in principle. Besides that, an acceptable accuracy of the image formation can be obtained only when the algorithms of a computed tomography are realized, what implies not only the usage of complex technical means, but a sufficiently high doze of an irradiation.

**Summary of the inventions**

A technical result, provided by the suggested inventions related to a method of radiotherapy of a malignant neoplasm, a method of defining of a placement of a malignant neoplasm, and a device for carrying out the said methods, resides in the elimination of the influence of the said factor owing to the usage of common X-ray beams both on defining the tissue structure and a placement of the malignant site, and on the ray action on the malignant site. Another type of the obtained technical result is decreasing the irradiation doze as a part of images formation of the tissues structure (the said images are used for correcting the irradiation program), as well as decreasing the irradiation doze of the tissues, surrounding the chosen area under ray acting.

The suggested method of radiotherapy of a malignant neoplasm with the use of X-ray beams, as well as the said known one, is realized in two stages. In the first stage an image of the internal structure of the part of the patient's body is formed, including a malignant neoplasm together with the organs and tissues surrounding the said part of the patient's body, on the basis of the information as a set of space coordinates of the points, the current results of measuring are referred to, and values of the tissues density, corresponding to the said coordinates. Then with the use of the results of the preceding diagnostics the images of the structural elements are identified, related to a malignant neoplasm, and an irradiation program is formed as a set of the X-ray dozes, which should be delivered to the different parts of the malignant neoplasm, represented by the fixed sets of the point coordinates. After that the second stage begins, when the formed irradiation program is realized.

To obtain the said types of the technical result in the suggested method, as against the known one, in the first stage to get the said information about the internal structure of the part of the patient's body an X-rays is concentrated in the zone with the point, the current results of measuring are referred to, placed inside the part of the patient's body, including a malignant neoplasm. A secondary radiation emerging in this zone is transported to one or more detectors, and then the part of the patient's body including a malignant neoplasm is scanned by relative moving the zone of the radiation concentration and the patient's body. On the basis of the set of values of the secondary radiation intensity, obtained by means of one or more detectors and defined simultaneously with the coordinates of the point of the X-ray concentration zone, the current results of measuring are referred to, the tissues density in this point is defined. The quantitative indexes, taking as the values of the tissues density, together with the values of coordinates, corresponding to the said quantitative indexes, are used for the imaging of the tissues density distributing in the part of the patient's body, including a malignant neoplasm. In the second stage a part of the space is scanned, occupied by a malignant neoplasm, by concentrating an X-rays by means of the same means as in the first stage so that the positions, occupied by the concentration zone, correspond to the parts of the malignant neoplasm, represented by the sets of the point coordinates, fixed in the first stage as a result of identifying of the images of the structural elements, related to the malignant neoplasm. The irradiation program, formed in the first stage, is realized by increasing the intensity of an X-rays in comparison with the first stage and controlling the irradiation time.

An X-rays concentrating in the zone with the point, the current results of measuring are referred to, placed inside the part of the patent's body, including a malignant neoplasm, can be realized, for instance, by means of one or more collimators with the use of corresponding quantity of spaced X-ray sources. Thus transporting of an emerging secondary radiation to one or more detectors can be realized as well by means of one or more collimators, thus all collimators should be oriented so that the axes of their central channels cross in the point, the current results of measuring are referred to.

It is possible as well to realize an X-rays concentrating in the zone with the point, the current results of measuring are referred to, placed inside the part of the patient's body, including a malignant neoplasm, by means of one or more X-ray half lenses, which transform a divergent radiation of the corresponding quantity of the spaced X-ray sources to a quasi-parallel one. Thus transporting of an emerging secondary radiation to one or more detectors is realized by means of one or more X-ray half lenses, focusing this radiation on the detectors or forming a quasi-parallel radiation, and all X-ray half lenses are oriented so that their optical axes cross in the point, the current results of measuring are referred to.

The X-rays concentrating in the zone with the point, the current results of measuring are referred to, placed inside the part of the patient's body, including a malignant neoplasm, can be realized as well by means of one or more X-ray half lenses, which transform a divergent radiation of the corresponding quantity of spaced X-ray sources to a quasi-parallel one, and transporting an emerging secondary radiation to one or more detectors is realized by means of one or more X-ray lenses, which focus this radiation on the detectors, thus all X-ray half lenses and lenses are oriented so that their optical axes cross in the point, the current results of measuring are referred to.

In one of the specific cases, when the suggested method is realized, an X-rays concentrating in the zone with the point, the current results of measuring are referred to, placed inside the part of the patient's body,

including a malignant neoplasm, is realized by means of more X-ray half lenses, which transform a divergent radiation of the corresponding quantity of spaced sources to a quasi-parallel one, and transporting an emerging secondary radiation to one or more detectors is realized by means of one or more collimators. Thus X-ray half lenses and collimators are oriented so that the central axes of all X-ray half lenses and the central channels of all collimators cross in the point, the current results of measuring are referred to.

In the other specific case an X-rays concentrating in the zone with the point, the current results of measuring are referred to, placed inside the part of the patient's body, including a malignant neoplasm, is realized by usage of one or more spaced X-ray sources and corresponding quantity of X-ray lenses, which focus a divergent X-rays of each source in the point, the current results of measuring are referred to. In this case transporting an emerging secondary radiation to one or more detectors is realized by means of X-ray lenses, which focus this radiation on the detectors and have the second focus in the said point. In this specific case an additional technical result is obtained, residing in the possibility of localizing the radiation action in the areas of supersmall sizes using small amounts of beams (even one) in combination with a low level of irradiating of healthy tissues, what makes possible to avoid fractioning of the irradiation and, in some cases, to carry out a radiotherapy of small tumors for one session. Such technical result can be obtained owing to the usage of X-ray lenses in the suggested invention.

In one more specific case an X-rays concentrating in the zone with the point, the current results of measuring are referred to, placed inside the part of the patient's body, including a malignant neoplasm, is realized by usage of one or more spaced X-ray sources and corresponding quantity of X-ray lenses, which focus the divergent radiation of each source in the point, the current results of measuring are referred to. Thus transporting an emerging secondary radiation to one or more detectors is realized by means of the collimators, oriented so that the optical axes of their central channels cross in the said point.

In the suggested method of determining a location of a malignant neoplasm with the use of X-ray beams, as well as in known one according to USA patent No. 5,207,223 [3], an image of the internal structure of the part of the patient's body is formed, including the surrounding organs and tissues, on the basis of the data as a set of space coordinates of the points, the current results of measuring are referred to, and the values of the tissues density, corresponding to the said coordinates. After that the images of the structural elements, related to the malignant neoplasm, are identified with the use of the results of the preceding diagnostics.

As against the said known one, in the suggested method to obtain the above mentioned technical result for receiving the said information about the internal structure of the part of the patient's body an X-rays is concentrated in the zone with the point, the current results of measuring are referred to, placed inside the part of the patient's body, including φ malignant neoplasm. A secondary radiation, emerging in this zone, is transported to one or more detectors; the part of the patient's body, including a malignant neoplasm, is scanned, by relative moving of the zone of a radiation concentration and the patient's body. The density of the biological tissues in the point, the current results of measuring are referred to, is determined on the basis of the set of the values of a secondary radiation, obtained by means of one or more detectors and determined simultaneously with the coordinates of the zone of an X-ray concentrating with the said point. The quantitative characteristics, taken to be the values of the density of the biological tissues, together with the coordinate values, corresponding to the said quantitative characteristics, are used to form the image of the density distribution of the biological tissues in the part of the patient's body, including a malignant neoplasm. Then the combination of the point coordinates and the densities of the biological tissues, corresponding to the said coordinates and identified as possessed by the malignant neoplasm, is fixed.

In the specific case of carrying out of the suggested method of determining a location of a malignant neoplasm an X-rays concentrating in the zone with the point, the current results of measuring are referred to, placed inside the part of the patient's body, including a malignant neoplasm, is realized by means of one or more collimators. Thus the corresponding quantity of the spaced X-ray sources is used, and transporting an emerging secondary radiation to one or more detectors is realized as well by means of one or more collimators; all collimators are oriented so that the axes of their central channels cross in the point, the current results of measuring are referred to.

In the other specific case an X-rays concentration in the zone with the point, the current results of measuring are referred to, placed inside the part of the patient's body, including a malignant neoplasm, is realized by means of one or more X-ray half lenses, which transform a divergent radiation of the corresponding quantity of the spaced X-ray sources to a quasi-parallel one, and transporting an emerging secondary radiation to one or more detectors is realized by means of one or more X-ray half lenses, which focus this radiation on the detectors or form a quasi-parallel radiation. Thus all X-ray half lenses are oriented so that their optical axes cross in the point, the current results of measuring are referred to.

In one more specific case an X-rays concentrating in the zone with the point, the current results of measuring are referred to, placed inside the part of the patient's body, including a malignant neoplasm, is realized by means of one or more X-ray half lenses, which transform a divergent radiation of the corresponding quantity of the spaced X-ray sources to a quasi-parallel one, and transporting an emerging secondary radiation to one or more detectors is realized by means of one or more X-ray lenses, which focus this radiation on the detectors. Thus all X-ray half lenses and lenses are oriented so that their optical axes cross in the point, the current results of measuring are referred to.

In the next specific case an X-rays concentrating in the zone with the point, the current results of measuring are referred to, placed inside the part of the patient's body, including a malignant neoplasm, is realized by means of several X-ray half lenses, which transform a divergent radiation of the corresponding quantity of the spaced X-ray sources to a quasi-parallel one, and transporting an emerging secondary radiation to one or more detectors is realized by means of one or more collimators. Thus X-ray half lenses and collimators are oriented so that the optical axes of all X-ray half lenses and the central channels of all collimators cross in the point, the current results of measuring are referred to.

An X-rays concentrating in the zone with the point, the current results of measuring are referred to, placed inside the part of the patient's body, including a malignant neoplasm, can be realized as well by means of one or more spaced X-ray sources and corresponding quantity of X-ray lenses, which focus a divergent X-rays of each source in the point, the current results of measuring are referred to, and transporting an emerging secondary radiation to one or more detectors is realized by means of X-ray lenses, which focus this radiation on the detectors and have the second focus in the said point.

Besides it is possible to concentrate an X-rays in the zone with the point, the current results of measuring are referred to, placed inside the part of the patient's body, including a malignant neoplasm, with the usage of one or more spaced X-ray sources and corresponding quantity of X-ray lenses, which focus a divergent X-rays of each source in the point, the current results of measuring are referred to. Transporting an emerging secondary radiation to one or more detectors in this case is realized by means of the collimators, oriented so that the optical axes of their central channels cross in the said point.

To realize both suggested methods one and the same device can be used. This device, as well as the known device according to the said USA patent No. 5,207,223 [3] for determining a location of a malignant neoplasm and its radiotherapy with the usage of X-ray beams, comprises an X-ray optical system, a means for the patient's body and the X-ray optical system relative positioning, a means for data processing and imaging. Thus the X-ray optical system comprises one or more X-ray sources with the means for their radiation concentrating and one or more detectors, which outputs are connected up to the means for data processing and imaging.

To obtain the above said types of the technical result, peculiar to the suggested inventions, in the suggested device as against the known one the X-ray sources, being a part of the X-ray optical system, are made with a capability of changing the intensity of their radiation, and the X-ray optical system comprises a means for mutual controlling of the radiation intensity of the X-ray sources. The means for radiation concentrating of these sources are made and placed with a capability of concentrating the radiation of all sources in the zone with the point, the current results of measuring are referred to, placed inside the part of the patient's body, including a malignant neoplasm. The X-ray optical system comprises as well one or more means for transporting a secondary radiation, emerging in the concentration zone, to the detectors, located at the outputs of these means, and the said detectors are made sensitive to the said secondary radiation. The sensors for determining the coordinates of the point, the current results of measuring are referred to, placed inside the part of the patient's body, including a malignant neoplasm, are connected with the means for the patient's body and the X-ray optical system relative positioning. The said sensors are connected with their outputs up to the means for data processing and imaging. The latter is made with a capability of forming and imaging the distribution of density of the tissues, resulting from scanning by the zone of the radiation of the X-ray sources concentrating the part of the patient's body, including a malignant neoplasm, by means of the means for the patient's body and the X-ray optical system relative positioning.

In one of the specific cases of carrying out the suggested device the X-ray optical system comprises several X-ray sources, and every means for concentrating the radiation of the said sources in the zone with the point, the current results of measuring are referred to, and each of the means for transporting a secondary radiation, emerging in the said point, is made as a collimator with the channels, oriented to the zone of concentrating the radiation of the X-ray sources, thus the optical axes of the central channels of all collimators cross in the point, the current results of measuring are referred to.

In this case it is possible, for instance, to use the quasi-pointed X-ray sources and the collimators with the channels, focused on these sources, as a part of the X-ray optical system, thus a screen with the holes is placed between the output of each X-ray source and the input of the corresponding collimator.

In the said case it is possible as well to use extended X-ray sources and the collimators with the channels, which widening toward these sources, as a part of the X-ray optical system.

In the other specific case the X-ray sources, being a part of the X-ray optical system, are quasi-pointed, each means for an X-rays concentrating in the zone with the point, the current results of measuring are referred to, is made as an X-ray half lens, which transforms a divergent radiation of the corresponding source to a quasi-parallel one, and each means for transporting an emerging secondary radiation to the detector is made as an X-ray half lens, which focuses this radiation on the detector. Thus the optical axes of all X-ray half lenses cross in the point, the current results of measuring are referred to.

In one more specific case the X-ray sources, being a part of the X-ray optical system, are quasi-pointed, each means for an X-rays concentrating in the zone with the point, the current results of measuring are referred to, is made as an X-ray half lens, which transforms a divergent radiation of the corresponding source to a quasi-parallel one, and each means for transporting an emerging secondary radiation to the detector is made as an X-ray half lens, which forms a quasi-parallel radiation and has a focus in the zone of an X-rays concentrating. Thus the optical axes of all X-ray half lenses cross in the point, the current results of measuring are referred to.

In the next specific case the X-ray sources, being a part of the X-ray optical system, are quasi-pointed, each means for an X-rays concentrating in the zone with the point, the current results of measuring are referred to, is made as an X-ray half lens, which transforms a divergent radiation of the corresponding source to a quasi-parallel one, and each means for transporting ans emerging secondary radiation to the detector is made as an X-ray lens, which focuses this radiation on the detector and has the second focus in the zone of X-rays concentration. In this case the optical axes of all X-ray half lenses and lenses cross in the point, the current results of measuring are referred to.

It is also possible to realize the device, when the X-ray sources, being a part of the X-ray optical system, are quasi-pointed, each means for an X-rays concentrating in the zone with the point, the current results of measuring are referred to, is made as an X-ray half lens, which transforms a divergent radiation of the corresponding source to a quasi-parallel one, and each means for transporting an emerging secondary radiation to the detector is made as a collimator with the channels, diverging toward the corresponding detector. Thus the optical axes of all X-ray lenses and half lenses and the central channels of the collimators cross in the point, the current results of measuring are referred to.

One more possibility of realizing the suggested device has the following peculiarity: the X-ray sources, being a part of the X-ray optical system, are quasi-pointed, each means for an X-rays concentrating in the zone with the point, the current results of measuring are referred to, is made as an X-ray half lens, which transforms a divergent radiation of the corresponding X-ray source to a quasi-parallel one, and each means for transporting an emerging secondary radiation to the detector is made as a collimator with the channels, converging toward the corresponding detector. Thus the optical axes of all X-ray half lenses and the central channels of the collimators cross in the point, the current results of measuring are referred to.

The other specific case of realizing the device, wherein the X-ray sources, being a part of the X-ray optical system, are quasi-pointed, each means for an X-rays concentrating in the zone with the point, the current results of measuring are referred to, is made as an X-ray lens, which focuses a divergent radiation of the X-ray source, and each means for transporting an emerging secondary radiation to the detector is made as an X-ray lens, which focuses this radiation on the corresponding detector. Thus the optical axes of all X-ray lenses cross in the point, the current results of measuring are referred to.

It is also possible to realize the suggested device in the following way: the X-ray sources, being a part of the X-ray optical system, are quasi-pointed, each means for an X-rays concentrating in the zone with the point, the current results of measuring are referred to, is made as an X-ray lens, which focuses a divergent radiation of the X-ray source, and each means for transporting an emerging secondary radiation to the detector is made as a collimator with the channels, converging toward the corresponding detector, the optical axis of all X-ray lenses and the central channels of the collimators cross in the point, the current results of measuring are referred to.

The suggested device can be realized as well so that the X-ray sources, being a part of the X-ray optical system, are quasi-pointed, and each means for an X-rays concentrating in the zone with the point, the current results of measuring are referred to, is made as an X-ray lens, which focuses a divergent radiation of an X-ray source. Thus each means for transporting an emerging secondary radiation to the detector is made as a collimator with the channels, diverging toward the corresponding detector; the optical axes of all X-ray lenses and the central channels of the collimators cross in the point, the current results of measuring are referred to.

In all described cases the device can be additionally completed with a means for switching off or screening the detectors for the work time of the X-ray sources with the increased intensity.

**The brief description of the figures**

The suggested inventions are illustrated with the figures:

fig. 1 depicts the principles, the suggested methods are based on: a diagram of the relative placement and joint of the main elements of a device for realizing the suggested methods;
fig. 2 and fig. 3 depict the specific cases of realizing the methods of carrying out the device with the usage of the collimators for an X-rays concentrating and transporting a secondary radiation to the detectors;
fig. 4 and fig. 5 depict the same with the usage of the X-ray half lenses;
fig. 6 depicts the same with the usage of the X-ray half lenses for an X-rays concentrating and the "full" X-ray lenses for transporting a secondary radiation to the detectors;

fig. 7 and fig. 8 depict the same with the usage of X-ray half lenses for an X-rays concentrating and the collimators for transporting a secondary radiation to the detectors;

fig. 9 depicts the same with the usage of X-ray lenses for an X-rays concentrating and transporting a secondary radiation to the detectors;

fig. 10 and fig. 11 depict the same with the usage of X-ray lenses for an X-rays concentrating and the collimators for transporting a secondary radiation to the detectors.

**Variants for carrying out the inventions**

The suggested method of determining a location of a malignant neoplasm is applied both as in its own right if a therapy of a malignant neoplasm does not follow it, and as a part of a method of radiotherapy of a malignant neoplasm in the first stage of carrying out the said method. In both cases this method, as such, is not diagnostic or therapeutic one.

The suggested method of radiotherapy of a malignant neoplasm always includes the suggested method of determining a location of a malignant neoplasm in the fist stage of its realization.

The suggested device is common for both methods.

The suggested methods are realized by means of the suggested device as follows.

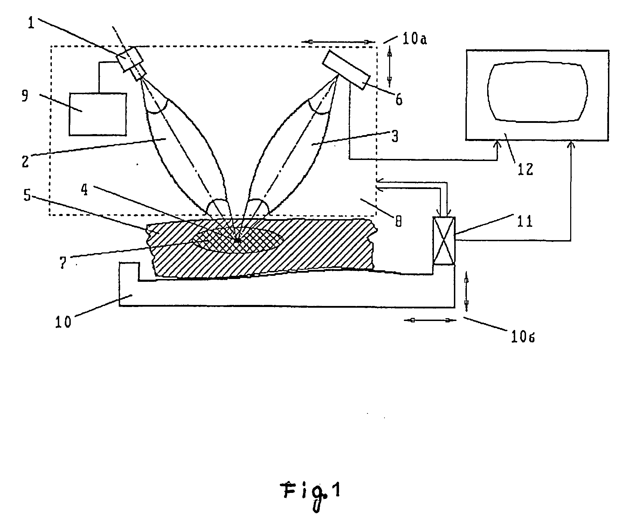

A divergent X-rays of the quasi-pointed source 1 (fig. 1) is focused by the X-ray lens 2 in the given point 4 of the part 7 of the patient's body 5, including a malignant neoplasm what results from the preceding diagnostics. The patient's body is placed in a required manner by means of a means 10 for the patient's body and the X-ray optical system relative positioning. A radiation, focused in the point 4, excites a secondary scattered radiation of the substance of the biological tissues of the patient 5 (coherent and incoherent Compton radiation, fluorescent radiation). The intensity of the secondary radiation accurate to the fluctuations, caused by the stochastic character of the process of the secondary radiation emerging, is proportional to the density of the substance, where it is formed. A focus of the second X-ray lens 3 is in the same point 4. This lens focuses the scattered secondary radiation, captured by it, on the detector 6, which transforms it to an electric signal, conducted to the input of the means 12 for data processing and imaging. A choice of a placement of the common focus point 4 of the lenses 1 and 3 is made by moving the patient's body 5 and the X-ray optical system 8 with respect to each other by means of the means 10 for their mutual positioning. The X-ray optical system 8 comprises the X-ray source 1, made with a capability of changing of the radiation intensity, the X-ray lenses 2, 3, and the radiation detector 6, as well as the means 9 for controlling the radiation intensity. The latter provides simultaneous changing of the radiation intensity of all sources involved in the X-ray optical system (only one of them is shown in the fig. 1, depicting the basic principles of the suggested inventions).

A possibility to change the radiation intensity and the means 9 for controlling the said intensity are used in the radiotherapy method in its second stage.

It should be explained that the X-ray lenses, being the means for X-rays controlling (a divergent radiation focusing, a quasi-parallel beam forming from the divergent radiation, a quasi-parallel beam focusing, etc.), represent a package of curved channels for radiation transporting, the radiation experienced a multiple total external reflection in (see, for instance: V.A. Arkadiev, A.I. Kolomiitsev, M.A. Kumakhov, et al. Broadband X-ray optics with wide angular aperture. Uspekhi fizicheskikh nauk, 1989, volume 157, issue 3, pp. 529-537 [4], where the first lens of this type is described, and USA patent No. 5744813 (published 28.04.98) [5], where more modern lens is described). A lens as a whole is barrel shaped (i.e. it narrows to both faces) if it is intended for a divergent radiation focusing, or it is half-barrel shaped (i.e. it narrows only to one face) if it is intended for a divergent radiation transforming to a quasi-parallel one or for such radiation focusing. The terms "a full lens" and "a half lens" are widely used to indicate the lenses of two said types.

Two variants of operation and usage of the device according to fig. 1 are possible. One variant represents an immovable patient's body 5, and the X-ray optical system 8 moves (the possibility of its movement is shown in fig. 1 by arrows 10a) with retention of the mutual arrangement of the elements 1, 2, 3, and 6 (and thus the focuses of the lenses 1 and 3 coincide). In the other variant, vice versa, the X-ray optical system 8 is immovable, and the patient's body 5 is moved (such movement is shown in fig. 1 by the arrows 10b).

The device comprises as well the coordinate sensor 11, which reacts to the mutual movement of the X-ray optical system 8 and the patient's body 5 and is connected to the means 10 for the patient's body and the X-ray optical system relative positioning. The sensor 11 must be adjusted so that its output signals correspond to the coordinates of the point, the current results of measuring are referred to with respect to the chosen reference point.

In the specific case, shown in fig. 1, the common focus point 4 of the X-ray lenses 2 and 3, their optical axes cross in, represents the said point, the current results of measuring are referred to.

In the other cases, when a zone of the radiation concentrating is more spread, such a point is a point of crossing the lines, being the optical axes (or taken conditionally as the optical axes, for instance, an axis of the central channel of the collimator) of a means for radiation concentrating and a means for transporting an emerging secondary radiation to the detectors. The means 10 for the patient's body and the X-ray optical system relative positioning should provide finding the said point in the limits of the part of the patient's body, being of interest and including (or hypothetically including) a malignant neoplasm.

A zone of a radiation concentrating represents an area of more or less sizes in relation to applied means for concentrating, and the said area surrounds the said point, the current results of measuring are referred to (in the second stage of carrying out a method of radiotherapy the concentration zone as well surrounds the point of crossing the lines, being the optical axes of the means for a radiation concentrating and the means for transporting an emerging secondary radiation to the detectors, however in this stage the measurements do not to carry out). In the case, shown in fig. 1, the size of the concentration zone is minimal.

The output signals of the sensor 11, as well as the output signal of the detector 6, are conducted to the inputs of the means 12 for data processing and imaging. As it was mentioned above, the focus point 4 represents in this case a point, the current results of measuring are referred to and in fact the radiation of the source 1 is concentrated in its surroundings (with regard to the finite size of the focus zone of the X-ray lens 2). The means 12 for data processing and imaging provides imaging the distribution of the substrate density of the biological tissues of the patient's body 5 and realizes one or other algorithm of two-dimensional or three-dimensional image forming on the screen (see, for instance: E. Lapshin. Graphics for IBM PC. Moscow, "Solon", 1995 [6]). In the simplest case, when, for example, scanning (moving the X-rays concentration zone with the point 4, the current results of measuring are referred to) is realized in any plane section of the patient's body 5, and image scan on the screen of the means 12 with long afterflow can be realized simultaneously with scanning. Storing of definite quantity of measuring results with following periodical image scan, etc., is possible as well. The capabilitie of digital equipment makes possible to form the image of density distributing in any plane section in the other variants of scanning of the area volume, including a malignant neoplasm, not only in the immediate section of interest. To do this it is sufficiently to choose such results from the obtained ones (a set of density values and the values of coordinates, corresponding to the density values), related to the volume, including the needed section, which correspond to the section of interest of the patient's body, to form and image their two-dimensional image with respect to the coordinate axes, placed in this section. The needed transformations of this type are realized by way of a programmer by means of the known methods, analogous to the described ones in [6].

To identify the structural elements of the formed image as related to the malignant neoplasm it is more appropriate just a scanning mode of the image, statistically stored in the digital form instead of the mode of the image analyzing in actual time at the scanning process.

The principle of functioning of the suggested inventions stems from the fact, that an intensity of an scattered secondary Compton radiation (a probability of quanta forming of this radiation), all other things being equal (in particular, at the given intensity of a primary X-rays, acting on the substance), is proportional to the substance density (see, for instance, J. Jackson. Classical electrodynamics. M., "Mir", 1965 [7]).

A main peculiarity of the suggested inventions is the usage of quanta of the scattered secondary Compton radiation as informing ones, as against the known methods and devices, when they are interference.

As it was mentioned, an important advantage in the medical applications is a capability of obtaining an acceptable accuracy at less radiation dozes, irradiating of the biological tissues.

To estimate a possible gain let's compare the suggested inventions with the most accurate of the modern methods of image forming of the invisible internal structure of the tissues and organs of a human's body, a computed X-ray tomography.

Let's take the following suggestions: photons energy is $E=50\ keV$, a zone of an X-rays concentrating is at 50 mm depth and is of $1\ mm\ x\ 1\ mm\ x\ 1\ mm$ sizes (such values are characteristic, for instance, for accuracy and observing conditions in mammography researches), the detector senses $5\%$ of the secondary radiation, emerging at the depth of $5\ cm$ (this suggestion means, that the secondary radiation, before it arrives the input of the means for the said radiation transporting to the detector, passes $5\ cm$ in the patient's body, thus a capture angle of a lens or a collimator, delivering the secondary radiation to the detector, is $0,05\ x\ 4\pi\ sr$). Taking into account that a linear coefficient of photon absorption in the patient's body is close to that one in the water and it is of $2\ x\ 10^{-1}\ 1/cm$ order at the energy $E=50\ keV$, the intensity of a primary beam of the radiation decreases in $exp(2\ x\ 10^{-1}\ x\ 5) = e \approx 2,71$ times, penetrating to the depth of 5 cm. Yielding from the patient's body the intensity of the secondary radiation (its photon energy is very close to $50\ keV$) decreases as well in $e \approx 2,71$ times. So, the total loss of the intensity is $e\ x\ e = 7,3$ times owing to the radiation absorption in the patient's body. Let's underrate the estimated gain and take into account only a Compton component of the secondary radiation. A probability of forming of quanta of the secondary Compton radiation at the depth of $\Delta_\chi$ is equal to $\omega = \sigma_k\ x\ N_e\ x\ \Delta_\chi$, where $\sigma_k = 6.55\ x\ 10^{-25}\ cm^2$ is a section of the secondary Compton scattering; $Ne = 3\ x\ 10^{23}\ 1/cm^3$ is the density of electrons in the water. So, at $\Delta_\chi = 1\ mm = 10^{-1}\ cm$ the probability is $\omega = 6.55\ x\ 10^{-25}\ x\ 3\ x\ 10^{23}\ x\ 10^{-1} \approx 2\ x\ 10^{-2}$. In other words, it is necessary in the average $1 : (2\ x\ 10^{-2}) = 50$ photons of the primary radiation to form one secondary photon at the length $\Delta_\chi = 1\ mm$.

Let's take an estimate error of the density (i.e. determining the quantity of secondary photons) of *1%* order. With regard to the random nature of process a root-mean-square value of a relative error is equal to $\delta = 1/(N)^{1/2}$, where *N* is a quantity of registered photons. *N = 10000* corresponds to $\delta = 0,01$.

So it is now possible to set up a simple equation for $N_\chi$, the needed quantity of primary photons, penetrating to the depth of *5 cm* and forming at this depth a secondary Compton radiation. The said radiation, in its turn, transmits *5 cm,* thus *N = 10000* photons reach the detectors:

$$N_\chi \, e^{-2} x \, 5x \, 10^{-2} \times 2 \times 10^{-2} = 10^4.$$

Here the coefficient *5 x $10^{-2}$* means that only *5% = $10^{-2}$* photons reach the detectors and are fixed from the total quantity of formed secondary photons.

Photons of *E = 50 keV* energy form an irradiation doze equal to 1 Roentgen, if the said photons flux is equal to *2,8 x $10^{10}$ 1/$cm^2$* (see the tabulated data for a relationship between a photon energy, their quantity and a doze, see, for example, Physics of image visualization in medicine. Ed. by S. Webb. M., "Mir", 1991 [8]). When it is suggested that a cross-section of the beam of the primary X-rays is equal to *1 $cm^2$* at the entrance in the patient's body, so a flux *7,3 x $10^7$ 1/$cm^2$* will form an irradiation doze equal to *2,6 x $10^{-3}$* Roentgen in the patient's body.

At the traditional X-ray computed tomography, for instance, at the osteoporosis research, an irradiation doze is usually equal to 100 ÷ 300 milliroentgen (V.I. Mazurov, E.G. Zotkin. Topical questions of diagnostics and treatment of osteoporosis. Saint-Petersburg, IKF "Foliant", 1998, p. 47 [9]), i.e. it is 100 times larger.

The doze can be additionally decreased in some times, if the irradiation is made by means of several sources, their beams reach the concentration zone in different paths and do not accumulate in the patient's body.

Therefore the most sufficient variants of carrying out the suggested methods and device, when several spaced X-ray sources and detectors with corresponding quantity of the means for a radiation concentrating and transporting a secondary Compton radiation to the detectors (lenses, half lenses, collimators) are used. On the one hand it makes possible to obtain more efficient concentration of a radiation (in the case of a single means for concentrating the said efficient concentration can be obtained only by usage of an X-ray lens as it is shown in fig. 1) and to increase a relationship signal/noise on the output of the detectors. On the other hand it makes possible to distribute the influence on the part of the patient's body under irradiation and to avoid overradation of the parts and organs, not to be studied. The usage of several detectors with simple averaging (or more complex processing of the output signals of different detectors in the means 12 for data processing and imaging, for example, "height" averaging or processing having regard to the density correlation in the points close to each other) makes possible to use X-ray sources of less power without the loss of precision at the other factors being equal. Besides that the influence of other factors, decreasing the accuracy, reduces at averaging (for example, distinct radiation absorption of the sources on the path to different points, a density is defined in, and a secondary radiation absorption on the path from this points to the inputs of the means for transporting a secondary Compton radiation to the detectors).

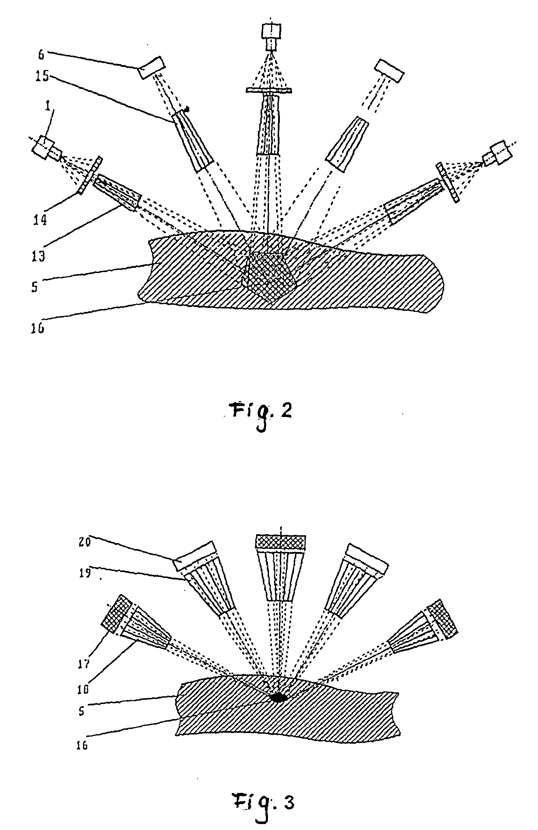

Such variants are given below (fig. 2 - fig. 11).

The simplest variants from the point of view of technical realization are shown in fig. 2 and fig. 3.

In the diagram in fig. 2 the quasi-pointed X-ray sources 1 and the collimators 13 with the channels, diverging (widening) toward the radiation propagation in order to concentrate it in the zone 16, are used. Between the sources 1 and the collimators 13 the screens 14 with the holes for the radiation transmitting to the inputs of the collimators and preventing its from direct (bypassing the collimators) fall on the object are placed. The secondary radiation is transported to the detectors 6 by means of the collimators 15 with the channels, converging (narrowing) toward the radiation propagation, i.e. toward the detectors 6, and the said collimators can have a focus on their sensitive surface. Semi-conducted detectors with narrow entrance aperture can be used as the detectors 6.

In fig. 3 the collimators have orientation just opposite the one, shown in fig. 2. It is sufficient to use extended X-ray sources 18 for total usage of the entrance aperture of the collimators 18, which concentrate the radiation in the zone 16. It is sufficient to use the detectors 20 with wide entrance aperture (for example, scintillation detectors) for the same reason.

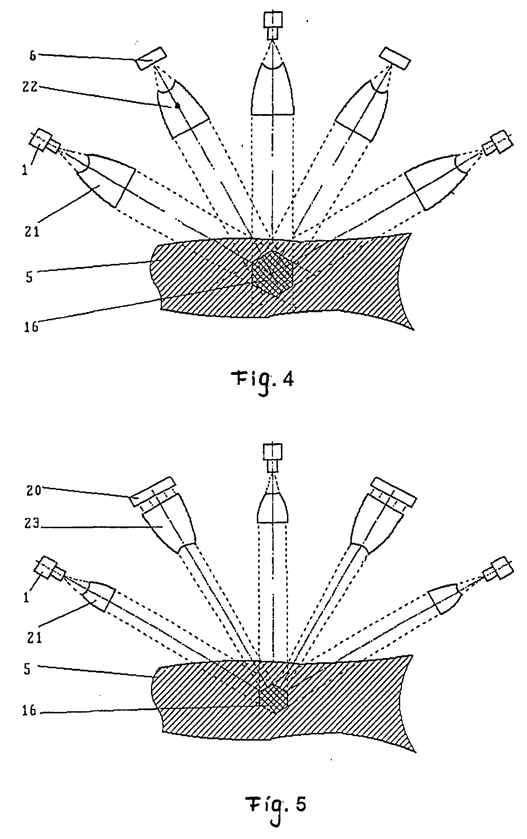

In fig. 4 the means for concentrating the radiation of the quasi-pointed sources 1 and the means for the secondary radiation transporting are made as the X-ray half lenses 21 and 22 correspondingly. Thus the half lenses 22 focus the scattered secondary radiation on the detectors 6.

In fig. 5 the means for concentrating the radiation of the quasi-pointed sources 1 and the means of transporting the secondary radiation are made as the X-ray half lenses 21 and 23 correspondingly. Thus the half lenses 23 transform the scattered secondary radiation to a quasi-parallel one and direct it on the detectors 20 with wide entrance aperture.

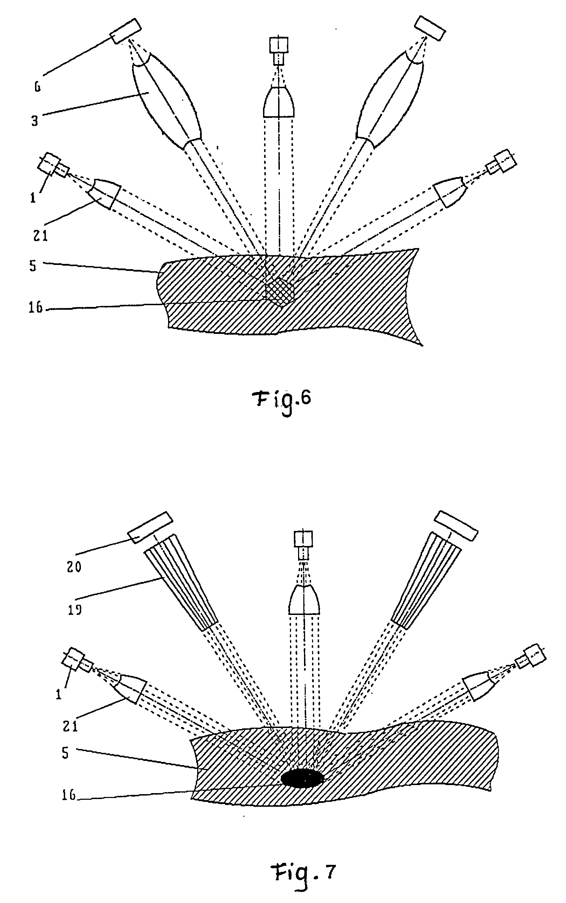

Fig. 6 depicts a combined variant: the means for concentrating the radiation of the quasi-pointed sources 1 are made as the X-ray half lenses 21, which direct the parallel beams to the zone 16, and the means for transporting the secondary Compton radiation to the detectors 6 are made as the "full" X-ray lenses 3.

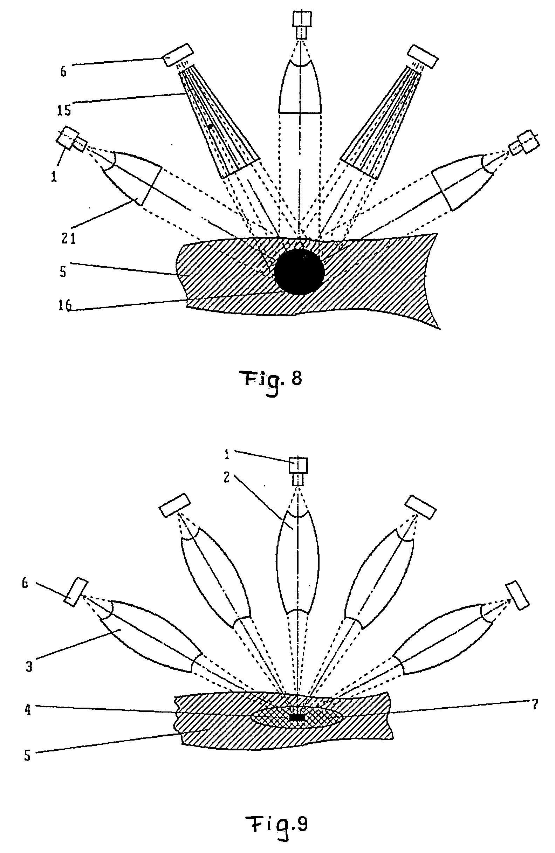

Fig. 7 and fig. 8 depict other combinations, wherein the means for transporting the secondary Compton radiation to the detectors are made as the collimators.

In fig. 7 the collimators 19 have channels, widening toward the detectors 6, and the latter have wide entrance aperture.

In fig. 8, just vice versa, the collimators 15 have the channels, narrowing toward the detectors 6, and the latter have narrow entrance aperture.

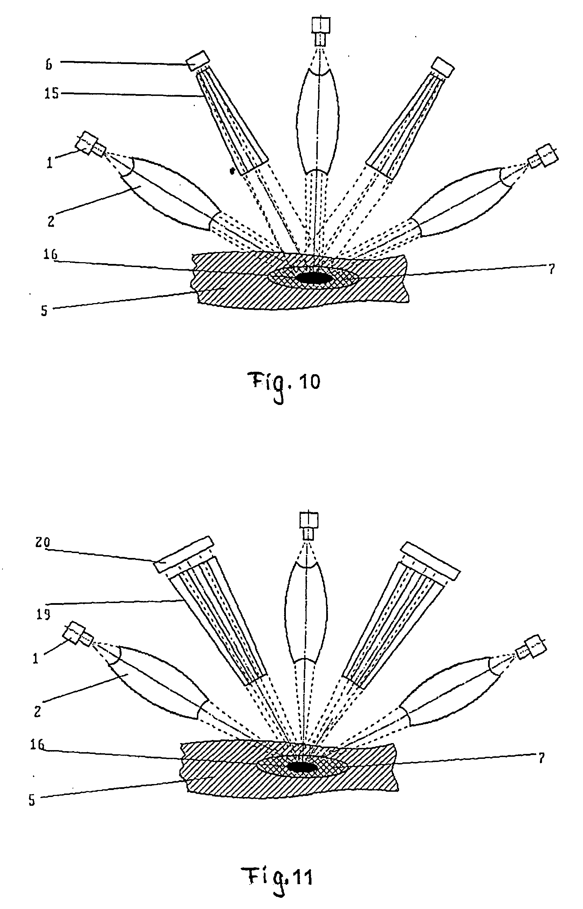

Fig. 9 depicts the most appropriate variant from the point of view of accuracy and resolution, where the means for concentrating the radiation of the quasi-pointed sources 1 and the means for transporting the secondary radiation to the detectors 6 are made as the "full" lenses 2 and 3 correspondingly (compare this variant with the one, shown in fig. 1).

Fig. 10 and fig. 11 depict two more combined variants. The fact that the "full" x-ray lenses 2 are used as the means for concentrating the radiation of the quasi-pointed sources 1 combines these variants.

Fig. 10 depicts the usage of the collimators 15, narrowing toward the detectors, as the means for transporting of the secondary radiation to the detectors 6 with narrow aperture.

Fig. 11 depicts the usage of the collimators 19, widening toward the detectors, as the means for transporting the secondary Compton radiation to the detectors 20 with wide aperture.

In all specific cases of the device embodying the mutual arrangement of the elements of the X-ray optical system 8 must eliminate falling the radiation of the sources (1, 17) directly or after transmission through the patient's body (5) on the inputs of the detectors (6, 20), as, as it was mentioned above, the secondary radiation, emerging in the concentration zone, carries the information about the density of the biological tissues under study. For this purpose no detector (and the means for transporting the secondary radiation to it) has to be on the continuation of the optical axis of any means for concentrating the source radiation in the concentration zone, representing an area of crossing the X-ray beams, formed by these means.

The suggested method of determining a location of a malignant neoplasm and the suggested device operating at carrying out the said method are finished by fixing the combinations of the point coordinates and the densities of the biological tissues corresponding to them, identified as belonging to the malignant neoplasm (for example, by storing the corresponding groups of digital codes in the means for data processing and imaging). Identification can by realized, for example, as in the known method [3] by way of comparing the images, resulting from carrying out the method, with those one, which resulted from the preceding diagnostics. Thus an operator, taking part in carrying out the method, can mark identified images of the structural elements on the screen of the means for data processing and imaging by means of traditional means for indicating of computer engineering, for example, "a mouse".

If a decision is taken to carry out a radiography of the malignant neoplasm, before the further usage of the device the irradiation program is formed as a set of dozes of an X-rays, which should be delivered to the different parts of the malignant neoplasm, represented by the fixed sets of the coordinates of the points. The irradiation program is formed with the usage of procedures, described, for example, in [1], in terms of the peculiarities of the organ, invaded by the malignant neoplasm, and other factors.

The irradiation program is realized by scanning the area, occupied by the malignant neoplasm, with the usage of the same means (the lenses 2, 21; the collimators 13, 18) for an X-rays concentrating, as in the first stage of carrying out the method of therapy, i.e. at realizing the method of determining the location of the malignant neoplasm. Thus the X-ray sources by means of the means 9 for a relative controlling of the radiation intensity of the said X-ray sources are switched on in each discrete position of the zone of radiation concentrating for the time, proportional to the required doze, at the increased level of the intensity (provided, for example, by increasing the anode current of the X-ray tubes), being sufficient for radiation injury of the tissues of the malignant neoplasm. In a specific case, if a malignant neoplasm is small, the irradiation can be carried out at a single position of the zone of X-rays concentrating, i.e. without scanning. Carrying out radiotherapy of microtumors (for example, an eye) is possible at the usage of the full lenses for the radiation concentrating.

The detectors can be switched off or screened mechanically for the time of the X-ray sources with increased radiation intensity operating to prevent possible breakdowns of the detectors (figures do not depict the said means).

The usage one and the same means for the radiation concentrating both at improving the location of the malignant neoplasm (in the first stage of the radiotherapy method) and at realizing the irradiation program (in the second stage) in combination with time distance of these stages minimize the errors of the radiation beams "focusing". The irradiation is carried out at the same positions of the zone of radiation concentrating, as in the stage of determining a location of a malignant neoplasm, as the X-ray optical system is placed with respect to the patient's body in positions, coinciding with the positions, fixed at identifying of the images of the structural elements as related to the malignant neoplasm. An accuracy of repeat positioning of the X-ray optical system relative to the patient's body in a position, corresponding to the coordinates, fixed at identifying, can be increased by using more perfect means for relative positioning, for example, as described in [2].

The usage of one or other logic of realization of the suggested methods and variants of the device embodiment is determined both by a probability of applying of such effective means for a radiation concentrating and transporting as the X-ray lenses and half lenses, and a required resolution. The latter affects on the choice of the parameters of the lenses and half lenses as well (such as a size of a focal spot, an extent of the focus region toward an optical axis of the lens, etc.). Thus it should be taken into account that realizing of high resolution at the usage of "full" lenses (parts of a millimeter order and higher) is connected with the increase of time, being sufficient for scanning the area, including the malignant neoplasm. Other conditions are taken into account as well, such as the availability of X-ray sources of proper power, sizes, etc.

An availability of described and numerous other variants of the suggested method realizing and the embodiment of the suggested device gives variety of possibilities for designing the means, meeting with the required particular requirements.

## Industrial Applicability

The suggested method of determining a location of a malignant neoplasm and its radiotherapy and a device for carrying out the said method are applied in terms of the diagnostics of the malignant neoplasm have being carried out and it is required to improve the data about its location, form, sizes. A therapy can by carried out as well by ray action, if a relevant decision was made before or is being made as a result of obtaining the said improved data.

## The information sources

1. Radiation therapy of malignant neoplasm. Physicians guide. Ed. by prof. E.S. Kiseleva. Moscow, "Medicina", 1996.
2. USA patent No. 5,983,424, published 16.11.1999.
3. USA patent No. 5,207,223, published 04.05.1993.
4. V.A. Arkadiev, A.I. Kolomiitsev, M.A. Kumakhov, et al. Broadband X-ray optics with wide angular aperture. Uspekhi fizicheskikh nauk, 1989, volume 157, issue 3.
5. USA patent No. 5744813, published 28.04.1998.
6. E. Lapshin. Graphics for IBM PC. M., "Solon", 1995.
7. G. Jackson. Classical electrodynamics. M., "Mir", 1965.
8. Physics of image visualization in medicine. Ed. by S. Webb. M., "Mir", 1991.
9. Topical questions of diagnostics and treatment of osteoporosis. Saint-Petersburg, IKF "Foliant", 1998.

## Claims

1. A method of radiotherapy of a malignant neoplasm with the usage of X-ray beams includes two stages: in the first stage an image of the internal structure of a part of a patient's body (5) is formed, including a malignant neoplasm, with the organs and tissues, surrounding it, on the basis of data as a set of space coordinates of the points, the current results of measuring are referred to, and the values of a density of biological tissues of the patient's body, corresponding to the said coordinates,

   the images of the structural elements, relating to the malignant neoplasm, are identified with the usage of the results of the preceding diagnostics,

   an irradiation program is formed as a set of X-ray dozes, which should be delivered to different parts of the malignant neoplasm, represented by fixed sets of the point coordinates,

   then the second stage begins, when formed irradiation program is carried out, wherein in the first stage to obtain the said data about the internal structure of the patient's body an X-rays is concentrated in the zone (16) with the point, the current results of measuring are referred to, placed inside the part of the patient's body, including the malignant neoplasm,

   a secondary radiation, emerging in the said zone, is transported to one or more detectors (6, 20),

   the part of the patient's body, including the malignant neoplasm, is scanned by mutual moving the zone of a radiation concentrating and the patient's body,

   on the basis of intensity values of the secondary radiation, obtained by means of one or more detectors and defined simultaneously with the coordinates of the point of the zone of X-rays concentrating, the current results of measuring are referred to, a density of the biological tissues in the said point is defined,

   the quantitative coefficients, taken as the values of a density of the biological tissues, with the values of coordinates, corresponding to the said coefficients, are used for forming the images of a density distribution of the biological tissues in the part of the patient's body, including the malignant neoplasm,

   in the second stage an area, occupied by the malignant neoplasm, is scanned, simultaneously concentrating an X-rays by means of the same means as in the first stage, so that the positions, occupied by the concentrating zone (16), correspond to the parts of the malignant neoplasm, represented by the sets of the point coordinates, fixed in the first stage as a result of the images of the structural elements identifying, related to the malignant neoplasm,

the irradiation program, formed in the first stage, is carried out by increasing an intensity of an X-rays as compared with the first stage and controlling an irradiation time.

2. A method according to claim 1, wherein an X-rays concentrating in the zone (16) with the point, the current results of measuring are referred to, placed inside the part of the patient's body, including the malignant neoplasm, is realized by means of one or more collimators (13, 18), using the corresponding quantity of spaced X-ray sources (1, 17),

transporting an emerging secondary radiation to one or more detectors is realized by means of one or more collimators (15, 19),

thus all collimators are oriented so that the axes of their central channels cross in the point, the current results of measuring are referred to.

3. A method according to claim 1, wherein an X-rays concentrating in the zone (16) with the point, the current results of measuring are referred to, placed inside the part of the patient's body, including the malignant neoplasm, is realized by means of one ore more X-ray half lenses (21), which transform a divergent radiation of corresponding quantity of spaced X-ray sources (1) to a quasi-parallel one,

transporting an emerging secondary radiation to one or more detectors is realized by means of one or more X-ray half lenses (22, 23), which focus this radiation on the detectors (6, 20) or form a quasi-parallel radiation,

thus all X-ray half lenses are oriented so that their optical axes cross in the point, the current results of measuring are referred to.

4. A method according to claim 1, wherein an X-rays concentrating in the zone (16) with the point, the current results of measuring are referred to, placed inside the part of the patient's body, including the malignant neoplasm, is realized by means of one or more X-ray half lenses (21), which transform a divergent radiation of corresponding quantity of spaced X-ray sources (1) to a quasi-parallel one,

transporting an emerging secondary radiation to one or more detectors is realized by means of one or more X-ray lenses (3), which focus this radiation on the detectors (6),

thus all X-ray half lenses and lenses are oriented so that their optical axes cross in the point, the current results of measuring are referred to.

5. A method according to claim 1, wherein an X-rays concentrating in the zone (16) with the point, the current results of measuring are referred to, placed inside the part of the patient's body, including the malignant neoplasm, is realized by means of several X-ray half lenses (21), which transform a divergent radiation of corresponding quantity of spaced sources to a quasi-parallel one,

transporting an emerging secondary radiation to one or more detectors is realized by means of one or more collimators (19, 15),

thus all X-ray half lenses and collimators are oriented so that the optical axes of all X-ray half lenses and the central channels of all collimators cross in the point, the current results of measuring are referred to.

6. A method according to claim 1, wherein an X-rays concentrating in the zone (4) with the point, the current results of measuring are referred to, placed inside the part of the patient's body, including the malignant neoplasm, is realized by usage of one or more spaced X-ray sources (1) and corresponding quantity of X-ray lenses (3), which focus a divergent X-rays of each source in the point, the current results of measuring are referred to,

transporting an emerging secondary radiation to one or more detectors (6) is realized by means of X-ray lenses (3), which focus this radiation on the detectors and have the second focus in the said point.

7. A method according to claim 1, wherein an X-rays concentrating in the zone (16) with the point, the current results of measuring are referred to, placed inside the part of the patient's body, including the malignant neoplasm, is realized by using one or more spaced X-ray sources (1) and corresponding quantity of X-ray lenses (2), which focus a divergent X-rays of each source in the point, the current results of measuring are referred to,

transporting an emerging secondary radiation to one or more detectors (6, 20) is realized by means of the collimators (15, 19), oriented so that the optical axes of their central channels cross in the said point.

8. A method of determining a location of a malignant neoplasm with the usage of X-ray beams, where an image of the internal structure of the part of the patient's body, including the malignant neoplasm and the organs and tissues, surrounding the said neoplasm, is formed on the basis of the information as a set of space coordinates of the points, the current results of measuring are referred to, and the values of density of the biological tissues, corresponding to that coordinates,

the images of the structural elements, related to the malignant neoplasm, are identified on the basis of the preceding diagnostics, wherein to obtain the said information about the internal structure of the part of the patient's body an X-rays is concentrated in the zone with the point, the current results of measuring are referred to, placed inside the part of the patient's body, including the malignant neoplasm,

a secondary radiation, emerging in the said zone, is transported to one or more detectors,

the part of the patient's body, including the malignant neoplasm, is scanned by relative moving the zone of a radiation concentrating and the patient's body,

on the basis of the set of values of the secondary radiation, obtained by means of one or more detectors and defined simultaneously with the coordinates of the point of the zone of X-rays concentrating, the current results of measuring are referred to, a density of the biological tissues in this point is defined,

the quantitative values, taken as the values of the density of the biological tissues, with the values of the coordinates, corresponding to the said quantitative values, are used to form the images of the density distribution of the biological tissues in the part of the patient's body, including the malignant neoplasm,

then the combinations of the coordinates of the points and the densities of the biological tissues, corresponding to the said points, identified as referred to the malignant neoplasm, are fixed.

9. A method according to claim 8, wherein an X-rays concentrating in the zone (16) with the point, the current results of measuring are referred to, placed inside the part of the patient's body (5), including the malignant neoplasm, is realized by means of one or more collimators (13, 18), using corresponding quantity of spaced X-ray sources (1, 17),

transporting an emerging secondary radiation to one or more detectors is realized as well by means of one or more collimators (15, 19),

thus all collimators are oriented so that the axes of their central channels cross in the point, the current results of measuring are referred to.

10. A method according to claim 8, wherein an X-rays concentrating in the zone (16) with the point, the current results of measuring are referred to, placed inside the part of the patient's body, including the malignant neoplasm, is realized by means of one or more X-ray half lenses (21), which transform a divergent radiation of corresponding quantity of spaced X-ray sources to a quasi-parallel one,

transporting an emerging secondary radiation to one or more detectors is realized by means of one or more X-ray half lenses (22), which focus this radiation on the detectors (6, 20) or form a quasi-parallel radiation,

thus all X-ray half lenses are oriented so that their optical axes cross in the point, the current results of measuring are referred to.

11. A method according to claim 8, wherein an X-rays concentrating in the zone (16) with the point, the current results of measuring are referred to, placed inside the part of the patient's body (5), including the malignant neoplasm, is realized by means of one or more X-ray half lenses (21), which transform a divergent radiation of corresponding quantity of spaced X-ray sources (1) to a quasi-parallel one,

transporting an emerging secondary radiation to one or more detectors (6) is realized by means of one or more X-ray lenses (22), which focus this radiation on the detectors,

thus all X-ray half lenses and lenses are oriented so that their optical axes cross in the point, the current results of measuring are referred to.

12. A method according to claim 8, wherein an X-rays concentrating in the zone (16) with the point, the current results of measuring are referred to, placed inside the patient's body, including the malignant neoplasm, is realized by means of several X-ray half lenses (21), which transform a divergent radiation of corresponding quantity of spaced sources (1) to a quasi-parallel one,

transporting an emerging secondary radiation to one or more detectors (6, 20) is realized by means of one or more collimators (15, 19),

thus the X-ray half lenses and collimators are oriented so that the optical axes of all X-ray half lenses and the central channels of all collimators cross in the point, the current results of measuring are referred to.

13. A method according to claim 8, wherein an X-rays concentrating in the zone with the point, the current results of measuring are referred to, placed inside the part of the patient's body, including the malignant neoplasm, is realized by usage of one ore more spaced X-ray sources (1) and corresponding quantity of X-ray lenses (2), which focus a divergent X-rays of each source in the point (4), the current results of measuring are referred to,

transporting an emerging secondary radiation to one or more detectors (6) is realized by means of the X-ray lenses (3), which focus this radiation on the detectors and have the second focus in the said point.

14. A method according to claim 8, wherein an X-rays concentrating in the zone with the point, the current results of measuring are referred to, placed inside the part of the patient's body, including the malignant neoplasm, is realized by usage of one or more spaced X-ray sources (1) and corresponding quantity of X-ray lenses (2), which focus a divergent radiation of each source in the point, the current results of measuring are referred to,

transporting an emerging secondary radiation to one or more detectors (6, 20) is realized by means of the collimators (15, 19), oriented so that the optical axes of their central channels cross in the said point.

15. A device for determining a location of a malignant neoplasm and its radiotherapy with the usage of X-ray beams, comprising an X-ray optical system (8), a device (10) for the patient's body and the X-ray optical system relative positioning, a device (12) for data processing and imaging, thus the X-ray optical system (8) includes one or more X-ray sources (1) with the devices (2) for their radiation concentrating and one or more detectors (6), which outputs are connected up to the device (12) for data processing and imaging, wherein the X-ray sources, being a part of the X-ray optical system (8), are made with a possibility of changing the intensity of their radiation,

the X-ray optical system comprises a means (9) of joint controlling by the radiation intensity of X-ray sources (1),

the means (2) for concentrating the radiation of these sources are made and placed with a possibility of concentrating the radiation of all sources in the zone with the point, the current results of measuring are referred to, placed inside the part of the patient's body (5), including the malignant neoplasm,

the X-ray optical system (8) comprises as well one or more means (3) for transporting a secondary radiation, emerging in the concentration zone, to the detectors (6), placed at the outputs of the said means and made sensitive to the said secondary radiation,

the sensors (11), connected with the means (10) of the patient's body and the X-ray optical system relative positioning, for determining the coordinates of the point, the current results of measuring are referred to, placed inside the part of the patient's body (5), including the malignant neoplasm, are connected with their outputs up to the means (12) for data processing and imaging,

the said means is made with a possibility of forming and imaging a density distribution of the tissues, resulting from scanning by the area of concentrating a radiation of the X-ray sources the part of the patient's body (5), including the malignant neoplasm, by means of the means (10) for the patient's body and the X-ray optical system relative positioning..

16. A device according to claim 15, wherein an X-ray optical system comprises several X-ray sources (1, 17),

each means for concentrating the radiation of the said sources in the zone (16) with the point, the current results of measuring are referred to, and each means for transporting a secondary radiation, emerging in the said zone, to the detectors (6, 20) are made as the collimators (13, 15, 18, 19) with the channels, oriented to the zone of concentrating a radiation of the X-ray sources,

thus the optical axes of the central channels of all collimators cross in the point, the current results of measuring are referred to.

17. A device according to claim 16, wherein the X-ray sources (1), being a part of the X-ray optical system, are quasi-pointed,

the collimators (13) have the channels, focused on the said sources,

a screen (14) with a hole is placed between the output of each X-ray source and the input of corresponding collimator.

18. A device according to claim 16, wherein the X-ray sources (17), being a part of the X-ray optical system, are extended,

the collimators (18) have the channels, widening toward the X-ray sources.

19. A device according to claim 15, wherein the X-ray sources (1), being a part of the X-ray optical system, are quasi-pointed,

each means for an X-rays concentrating in the zone with the point, the current results of measuring are referred to, is made as an X-ray half lens (21), which transforms a divergent radiation of the corresponding source to a quasi-parallel one,

each means for transporting an emerging secondary radiation to the detector is made as an X-ray half lens (22), focusing this radiation on the detector (6),

thus the optical axes of all X-ray half lenses cross in the point, the current results of measuring are referred to.

20. A device according to claim 15, wherein the X-ray sources (1), being a part of the X-ray optical system, are quasi-pointed,

each means for X-rays concentrating in the zone with the point, the current results of measuring are referred to, is made as an X-ray half lens (21), which transforms a divergent radiation of the corresponding source to a quasi-parallel one,

each means for transporting an emerging secondary radiation to the detector (20) is made as an X-ray half lens (23), which forms a quasi-parallel radiation and has a focus in the zone (16) of an X-rays concentrating,

thus the optical axes of all X-ray half lenses cross in the point, the current results of measuring are referred to.

21. A device according to claim 15, wherein the X-ray sources (1), being a part of the X-ray optical system, are quasi-pointed,

each means for X-rays concentrating in the zone (16) with the point, the current results of measuring are referred to, is made as an X-ray half lens (21), which transforms a divergent radiation of the corresponding source to a quasi-parallel one,

each means for transporting an emerging secondary radiation to the detector (20) is made as an X-ray lens (3), which focuses this radiation on the detector (6) and has the second focus in the zone of an X-rays concentrating,

the optical axes of all X-ray half lenses and lenses cross in the point, the current results of measuring are referred to.

22. A device according to claim 15, wherein the X-ray sources (1), being a part of the X-ray optical system, are quasi-pointed,

each means for an X-rays concentrating in the zone with the point, the current results of measuring are referred to, is made as an X-ray half lens (21), which transforms a divergent radiation of the corresponding source to a quasi-parallel one,

each means for transporting an emerging secondary radiation to the detector is made as a collimator (19) with the channels, diverging toward the corresponding detector (20),

the optical axes of all X-ray lenses and half lenses and the central channels of the collimators cross in the point, the current results of measuring are referred to.

23. A device according to claim 15, wherein the X-ray sources (1), being a part of the X-ray optical system, are quasi-pointed,

each means for an X-rays concentrating in the zone (16) with the point, the current results of measuring are referred to, is made as an X-ray half lens (21), which transforms a divergent radiation of the corresponding X-ray source to a quasi-parallel one,

each means for transporting an emerging secondary radiation to the detector is made as a collimator (15) with the channels, converging toward the corresponding detector (6),

the optical axes of all X-ray half lenses and the central channels of the collimators cross in the point, the current results of measuring are referred to.

24. A device according to claim 15, wherein the X-ray sources (1), being a part of the X-ray optical system, are quasi-pointed,

each means for an X-rays concentrating in the zone with the point, the current results of measuring are referred to, is made as an X-ray lens (2), which focuses a divergent radiation of an X-ray source,

each means for transporting an emerging secondary radiation to the detector is made as an X-ray lens (3), which focuses this radiation on the corresponding detector (6),

the optical axes of all X-ray lenses cross in the point (4) the current results of measuring are referred to.

25. A device according to claim 15, wherein the X-ray sources (1) being a part of the X-ray optical system are quasi-pointed,

each means for an X-rays concentrating in the zone with the point, the current results of measuring are referred to, is made as an X-ray lens (2), which focuses a divergent radiation of an X-ray source,

each means for transporting an emerging secondary radiation to the detector is made as a collimator (15) with the channels, converging toward the corresponding detector (6),

the optical axes of all X-ray lenses and the central channels of the collimators cross in the point, the current results of measuring are referred to.

26. A device according to claim 15, wherein the X-ray sources (1) being a part of the X-ray optical system are quasi-pointed,

each means for an X-rays concentrating in the zone (16) with the point, the current results of measuring are referred to, is made as an X-ray lens (2), which focuses a divergent radiation of an X-ray source,

each means for transporting an emerging secondary radiation to the detector is made as a collimator (19) with the channels, diverging toward the corresponding detector (20),

the optical axes of all X-ray lenses and the central channels of the collimators cross in the point, the current results of measuring are referred to.

27. A device according to any one of claims 15-26, wherein the said device comprises additionally the means for switching off or screening the detectors for the time of the X-ray sources operation with the increased intensity.

Fig. 1

Fig. 2

Fig. 3

Fig. 4

Fig. 5

Fig.6

Fig. 7

Fig. 8

Fig.9

Fig. 10

Fig. 11

## INTERNATIONAL SEARCH REPORT

| | |
|---|---|
| International application No. | |
| PCT/RU 00/00273 | |

**A. CLASSIFICATION OF SUBJECT MATTER**

A61N 5/10, A61B 6/00, G01N 23/04

According to International Patent Classification (IPC) or to both national classification and IPC

**B. FIELDS SEARCHED**

Minimum documentation searched (classification system followed by classification symbols)

A61N 5/00, 5/10, A61 B6/00-6/14, A61B 5/05, G01N 23/00-23/04, G21K 1/00-1/06, G21K 5/00-5/10

Documentation searched other than minimum documentation to the extent that such documents are included in the fields searched

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| | US 5207223 A ( ACCURAY, INC. )  4 May 1993 (4.05.1993) | |
| X | The abstract, figures 1, 3, 4 | 15 |
| Y | The whole document | 16-26 |
| A | | 1-14, 27 |
| | US 5528650 A ( STUART SWERDLOFF et al )  18 June 1998 (18.06.1998) | |
| Y | The abstract, figures 1, 4, 9 | 17 |
| A | | 1,2, 7-9 |
| | US 5596619 A ( NOMOS CORPORATION )  21 January 1997 (21.01.1997) | |
| Y | The abstract | 19 |
| A | Figures 1, 6,10, 15-18 | 1, 3, 8, 10 |
| | US 5627870 A (ATEA, SOCIETE ATLANTIQUE DE TECHNIQUES AVAN- | |

☒ Further documents are listed in the continuation of Box C.   ☐ See patent family annex.

| | |
|---|---|
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "E" earlier document but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | |
| "O" document referring to an oral disclosure, use, exhibition or other means | "Y" document of particular relevance; the claimed invention cannot be considered to involve an inventive step when the document is combined with one or more other such documents, such combination being obvious to a person skilled in the art |
| "P" document published prior to the international filing date but later than the priority date claimed | "&" document member of the same patent family |

| Date of the actual completion of the international search | Date of mailing of the international search report |
|---|---|
| 21 February 2001 (21.02.2001) | 08 February 2001 (08.02.2001) |

| Name and mailing address of the ISA/ | Authorized officer |
|---|---|
| RU | |
| Facsimile No. | Telephone No. |

Form PCT/ISA/210 (second sheet) (July 1992)

22

## EP 1 195 177 A1

| Category* | Citation of document, with indication, where appropriate, of the relevant passages | Relevant to claim No. |
|---|---|---|

**INTERNATIONAL SEARCH REPORT**

International application No.

PCT/RU 00/00273

**C (Continuation). DOCUMENTS CONSIDERED TO BE RELEVANT**

| Category* | Citation of document, with indication, where appropriate, of the relevant passages | Relevant to claim No. |
|---|---|---|
| Y | CEES ) 6 May 1997 (06.05.1997) The abstract, figures 1-3 | 16, 18 |
| A | | 2-7, 9-14 |
| Y | WO 96/ 01991 A1 (KUMAKHOV Muradin Abubekirovich) 25 January 1996 (25.01.1996), the claims, figures 1-33, the abstract | 19-26 |

Form PCT/ISA/210 (continuation of second sheet) (July 1992)

23